# EUROPEAN PATENT SPECIFICATION

(11) **EP 4 031 333 B1**
(45) Date of publication and mention of the grant of the patent: **19.02.2025**
(21) Application number: 20789259.7
(22) Date of filing: 16.09.2020
(51) Int. Cl.: B25J 9/10, B25J 15/00, F16H 1/32, A61F 2/68

(54) **ACTUATOR FOR ROBOTIC DEVICES**
BETÄTIGUNGSVORRICHTUNG FÜR ROBOTER
ACTIONNEUR POUR DISPOSITIFS ROBOTISÉS

(30) Priority: 18.09.2019 IT 201900016631
(43) Date of publication of application: 27.07.2022
(73) Proprietor: Fondazione Istituto Italiano di Tecnologia, 16163 Genova (IT); INAIL - Istituto Nazionale per l'Assicurazione contro gli Infortuni, 00187 Roma (IT)
(72) Inventor: ANDREA, Lince, 15064 Fresonara (AL) (IT); SAMUEL, Stedman, 16145 GENOVA (GE) (IT); LORENZO, Lombardi, 16149 Genova (GE) (IT); NICOLÒ, Boccardo, 16159 Genova (GE) (IT); SIMONE, Traverso, 16165 Genova (GE) (IT); PAOLO, Rossi, 16165 Genova (GE) (IT); MATTEO, Laffranchi, 16128 Genova (GE) (IT); LORENZO, De Michieli, 16148 Genova (GE) (IT)
(74) Representative: Lunati & Mazzoni S.r.L.
(86) International application number: PCT/IB2020/058618
(87) International publication number: WO 2021/053539

(56) References cited:
- JP-A- 2014 163 440
- US-A1- 2012 325 040
- US-A1- 2016 106 615

## Description

This invention relates to an actuator for robotic devices of the type specified in the preamble of the first claim.

In particular, the invention concerns an actuator designed to be used for a robotic device e.g. anthropomorphic (such as those typical of the prosthetics sector for controlling a prosthesis such as that of an artificial prosthetic/myoelectric hand capable of reproducing the movements of a human hand) or non-anthropomorphic (e.g. suitable for material-handling or pick and place movements), such as grippers. As is well known, anthropomorphic devices and, in particular, prostheses (such as artificial limbs, artificial hands, hereinafter simply referred to as hands) consist of artificial devices that reproduce a part of a body (animal or especially human) such as a robot, a robotic arm, and, preferably, a device replacing a missing part of the body and, more preferably, a prosthetic hand.

Prostheses are mainly divided into passive and active ones.

Passive prostheses are used to replace an amputated body part in order to restore bodily integrity with particular attention to aesthetics. These prostheses are characterised by a stiff, non-motorised structure. They are not able to grip things. Active prostheses, and especially hands, have mechanical and/or electronic components that, by articulating the parts forming the prosthesis, can reproduce poses/grips; and one or more actuators designed to control the motion of said mechanical components usually through one or more cables moved by the actuator. Specifically, active prostheses can include a number of actuators equal to the number of degrees of freedom or be under-implemented and, therefore, have fewer actuators than the number of degrees of freedom.

Non-anthropomorphic devices and, in particular, industrial robots (e.g. robotic arms, grippers, end effectors, or other similar devices designed to interact with the environment and mostly used to handle/manipulate an object or perform an operation usually in an industrial environment) have actuators similar to those of active prostheses.

The actuators of non-anthropomorphic devices therefore consist of mechanical/electronic components that, by articulating the parts, enable the device to manipulate an object.

Well known actuators basically consist of motors, usually servomotors, to the output shaft of which a pulley is attached, which controls an at least partial actuation cable of the prosthesis. Examples of actuators are described in US2016106615A1, US2012325040A1, JP2014163440A, WO2007076763, WO201411184, EP3457995, and EP0045818.

The prior art described here has a few major drawbacks.

In fact, the prior art described above and, in particular, the patents mentioned, having only one motor, are only able to perform a limited number of poses and grips compared to those of a prosthesis (in the case of anthropomorphic devices) or few manipulations compared to those performed manually by an operator.

It should be noted that this problem is extremely significant in under-implemented hands. In fact, the prosthetic hands described in the patents mentioned, having a single motor, have reduced actuation flexibility. This translates into reduced comfort of use, making a robotic hand, especially when used in the prosthetics sector, unlikely to meet a patient's expectations.

It should be emphasised that the existence of solutions in which the prosthesis and, specifically, the under-implemented hand has several motors, usually two, working in opposition or on different kinematics each of which control the motion of part of the fingers. These solutions, while improving the usability of the prosthesis, are extremely bulky and are, therefore, hardly preferable to a single-implemented robotic prosthesis.

The above-mentioned drawback is also significant in the industrial field where the limited number of manipulations requires the purchase of a large number of devices. It should be noted that, in recent years, industrial devices have been developed that are capable of performing a good number of operations, but which are characterised by complicated mechanics that make them difficult to use and increase the costs and risk of failure thereof.

In this situation, the technical task underlying this invention is to devise a prosthetic actuator capable of basically overcoming at least part of the above-mentioned drawbacks.

In the field of said technical task, it is an important purpose of the invention to obtain an actuator that is simple to construct that allows a robotic device (anthropomorphic or non-anthropomorphic) to perform a large number of poses/manipulations. Another important purpose of the invention is to provide a robotic device that is compact in size.

In addition, in the case of anthropomorphic robotic devices, an important purpose of the invention is to make a prosthesis characterised by great comfort of use and, therefore, one that fully satisfies a patient's expectations. In the case of non-anthropomorphic robotic devices it is important to obtain a robotic device with simple mechanics, which is, therefore, easy to use and economical.

The technical task and the specified purposes are achieved with an actuator as claimed in the appended claim 1. Preferred embodiments are described in the dependent claims.

The features and advantages of the invention are clarified below by the detailed description of preferred embodiments of the invention, with reference to the accompanying drawings, wherein:
**Fig. 1** shows, in scale, a robotic device, in the particular, non-limiting example of an anthropomorphic device, equipped with an actuator according to the invention;

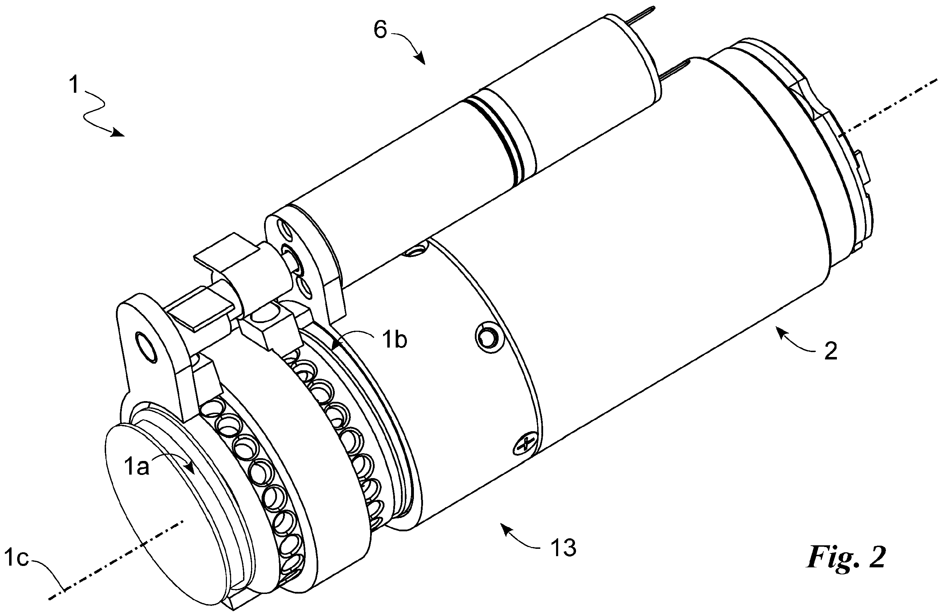

**Fig. 2** illustrates, to scale, an actuator according to the invention;
**Fig. 3** presents, to scale, a cross-section of the actuator;
**Fig. 4** is, to scale, a cross-section of the actuator in a different configuration;

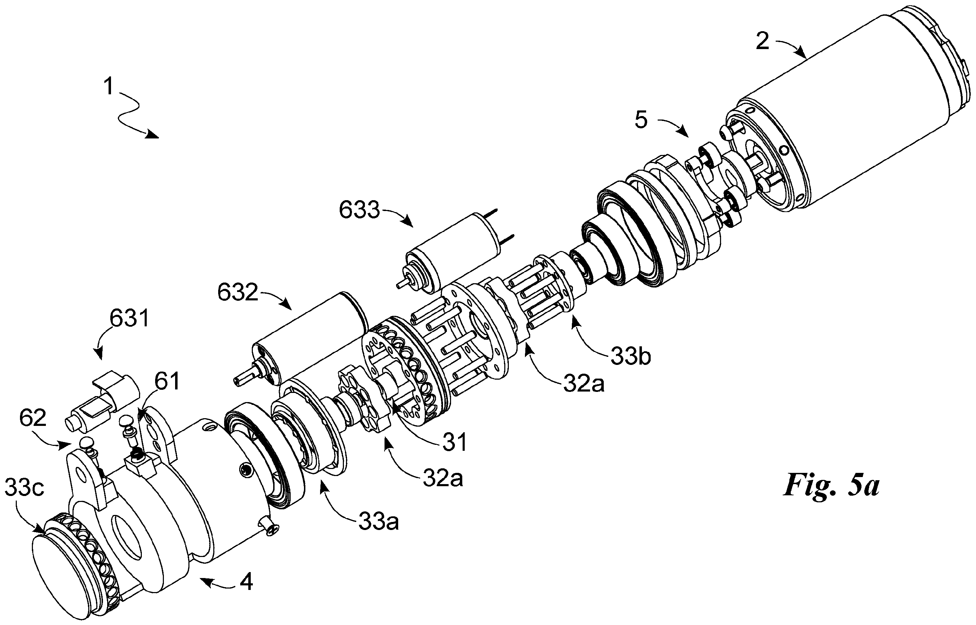

**Fig. 5a** shows, to scale, an exploded view of the actuator according to the invention;

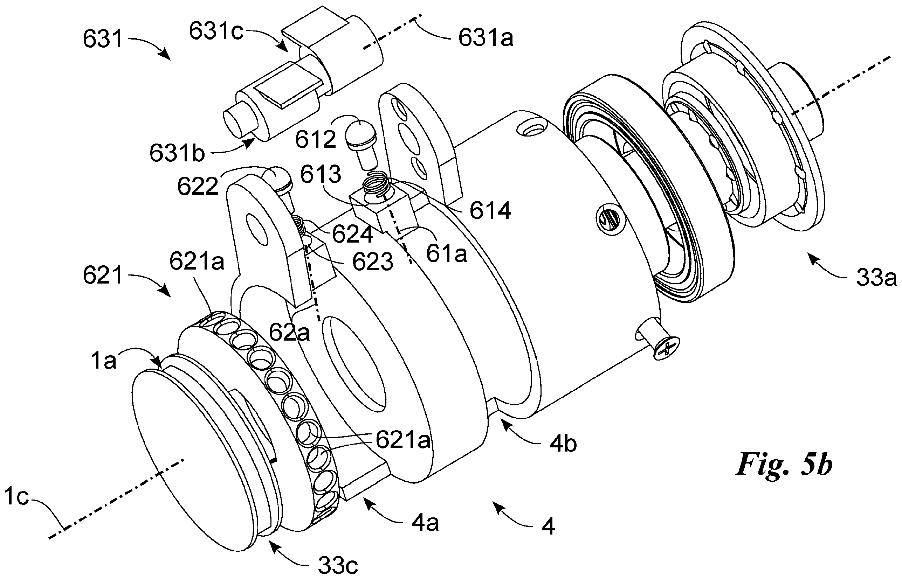

**Fig. 5b** illustrates, to scale, an exploded view of the assembly in Fig. 5a;

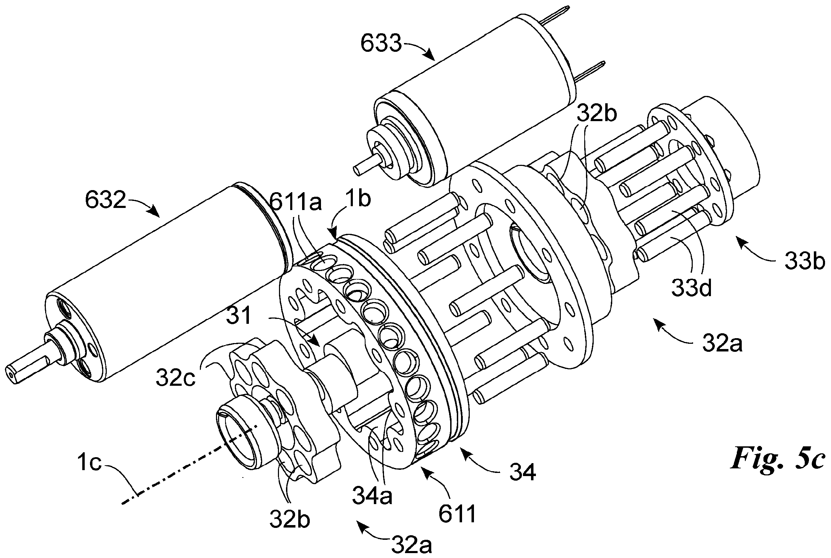

**Fig. 5c** highlights, to scale, a second assembly of the exploded view in Fig. 5a;

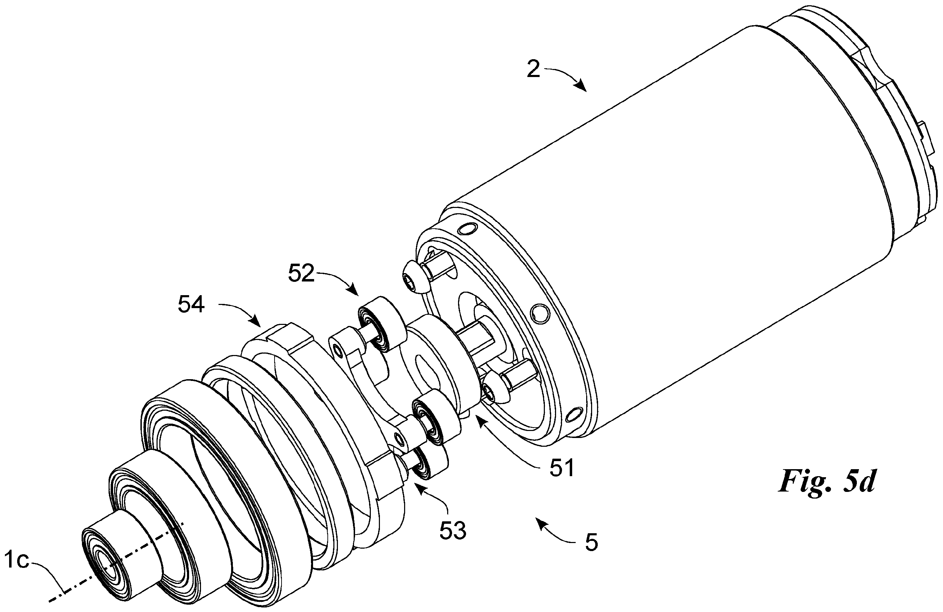

**Fig. 5d** shows, to scale, a third assembly of the exploded view in Fig. 5a;

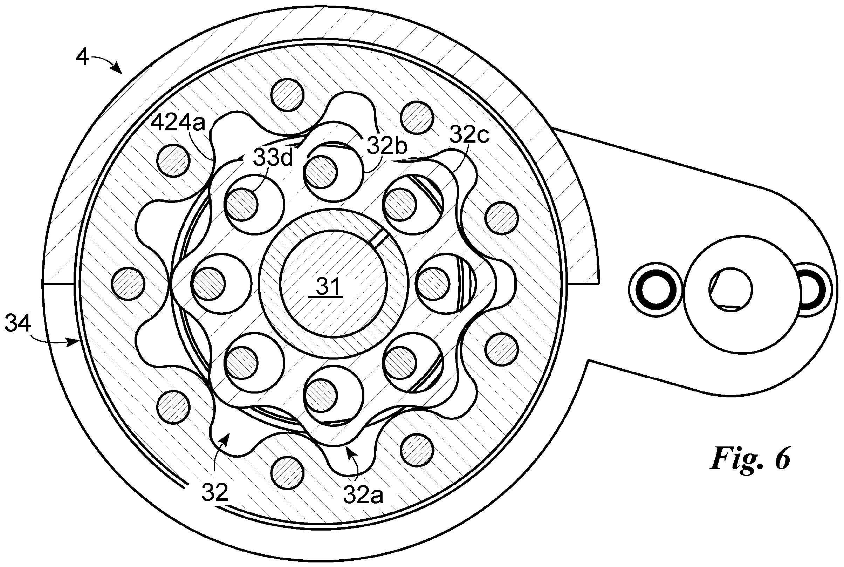

**Fig. 6** illustrates, to scale, a different cross-section of the actuator; and

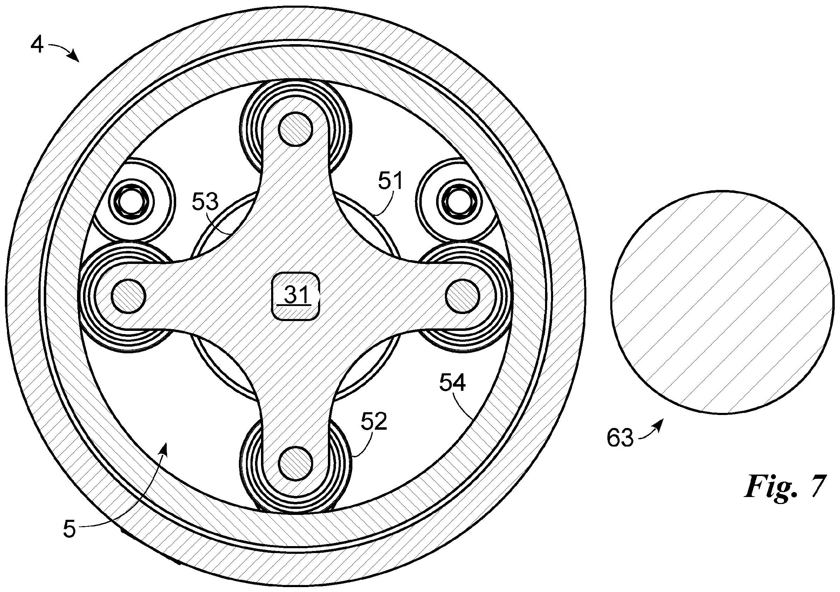

**Fig. 7** shows, to scale, an additional cross-section of the actuator.

In this document, the measures, values, shapes and geometric references (such as perpendicularity and parallelism), when used with words like "about" or other similar terms such as "approximately" or "substantially", are to be understood as except for measurement errors or inaccuracies due to production and/or manufacturing errors and, above all, except for a slight divergence from the value, measure, shape or geometric reference with which it is associated. For example, if associated with a value, such terms preferably indicate a divergence of no more than 10% from the value itself.

Furthermore, when terms such as "first", "second", "upper", "lower", "main" and "secondary" are used, they do not necessarily identify an order, relationship priority or relative position, but they can simply be used to distinguish different components more clearly from one another.

Unless otherwise stated, the measurements and data contained in this text shall be considered as performed in ICAO International Standard Atmosphere (ISO 2533).

Unless otherwise specified, as is apparent from the following discussions, terms such as "processing", "computing", "determination", "calculation", or similar, are considered to refer to the action and/or processes of a computer or similar electronic calculation device, which manipulates and/or transforms data represented as physical, such as electronic magnitudes of registers of an IT system and/or memories, other data similarly represented as physical quantities within IT systems, registers or other information memorization, transmission or display devices.

With reference to the figures, the reference number **1** globally denotes the actuator according to the invention.

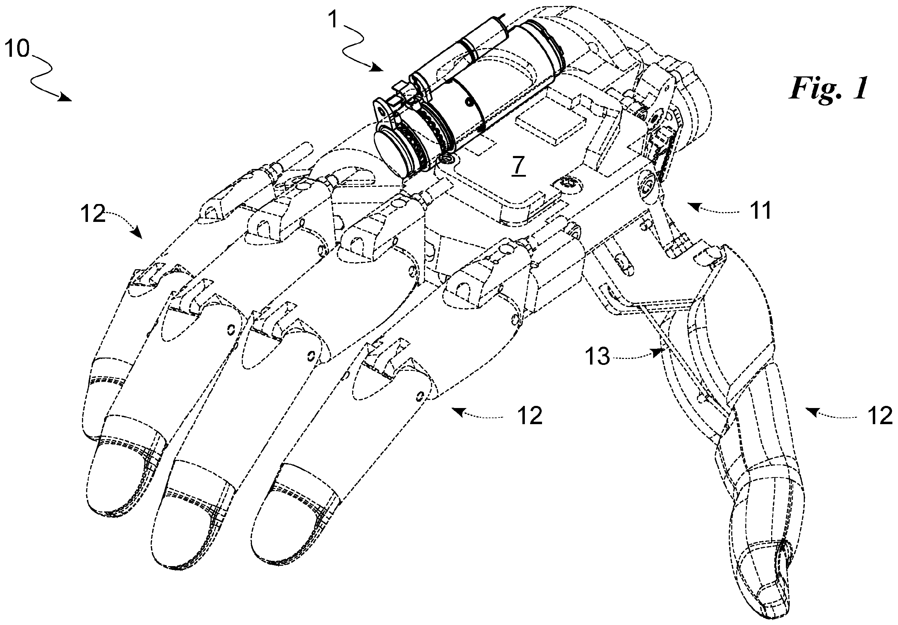

It is designed to be used to control the movements of a (preferably a single) robotic device **10,** preferably consisting of a manipulator, for example an anthropomorphic or non-anthropomorphic manipulator. For example, the robotic device 10 can be a robotic hand or, preferably, a prosthesis and, more preferably, a limb or a hand as illustrated in Fig. 1.

The robotic device 10 may comprise, at least one actuator 1, a base body **11** to support at least one actuator 1; one or more mobile components **12** designed to be moved; a transmission kinematic mechanism **13** designed to receive the motion from the actuator 1 and to transmit it to one or more components.

The robotic device 10 may comprise, in addition, a connection designed to enable the robotic device 10 to be attached to the patient's body.

The robotic device 10, in the non-limiting example in Fig. 1, may consist of a prosthesis and, specifically, a prosthetic hand, conveniently under-implemented, capable of reproducing the movements of a human hand. Said hand and, therefore, the robotic device 10 may comprise at least one finger (preferably five fingers); a base body 11; and at least one transmission kinematic mechanism 13 designed to receive the motion from the actuator 1 and transmit it to one or more fingers.

The actuator is designed to be attached integrally to the robotic device 10. For example, in the case of a hand, it is integral with the base body 11.

In this non-limiting example, the base body 11 is designed to be attached to an external element such as a robotic arm or a human limb. The base body 11 defines a main extension surface. It consists of the palm of the under-implemented hand 1. Each finger may comprise one or more phalanges, each of which consists of a mobile component 12. Specifically, one finger may comprise one phalange (i.e. a first mobile component 12) proximal to the base body 11 and one phalange (i.e. a second mobile component 12) distal to the base body 11; and in some cases an intermediate phalange (i.e. a third mobile component 12) having an end hinged to the proximal phalange and the opposite end hinged to the distal phalange.

An example of a finger is described in PCT/IB2019/053682.

The actuator 1 defines at least one motion output and, in particular, a first output **1a** and a second output **1b.**

The outputs 1a and 1b preferably define, between them, a motion of a different modulus (for example different speed and\or acceleration) and, specifically, the same direction.

The transmission kinematic mechanism 13 is designed to control the actuation of the robotic device 10 based on the output motion of the actuator 1, i.e. from the outputs 1a and 1b.

The transmission kinematic mechanism 13 is integral with the base body 11.

In some cases, the robotic device 10 may comprise several transmission kinematic mechanisms 13 designed to control the motion of components of the robotic device itself, preferably independently of each other.

For example, an under-implemented hand may comprise a first transmission kinematic mechanism 13 designed to receive the motion from the first output 1a and controlling a first part of the mobile components 12; and a second transmission kinematic mechanism 13 designed to receive the motion from the second output 1b and controlling a second part of the mobile components 12 at least to complement said first part.

Alternatively, the under-implemented hand may comprise just one transmission kinematic mechanism 13 actuating all the mobile components 12. Conveniently, the outputs 1a and 1b define, between them, a motion of a different modulus and, specifically, the same direction.

An example of a transmission kinematic mechanism 13 is described in PCT/IB2019/053682. Unlike what is described in PCT/IB2019/053682, it should be noted that the robotic device 10 (in this case an under-implemented hand) comprises a first transmission cable to transmit motion from the first output 1a to the first control cable described in PCT/IB2019/053682 and a second transmission cable designed to transmit motion from the second output 1b to said first control cable. The transmission kinematic mechanism 13 can, therefore, comprise a connection device designed to enable each transmission cable to control the first control cable and, therefore, the mobile components 12 as described in PCT/IB2019/053682.

The actuator 1 defines a longitudinal axis 1c conveniently barycentric to the actuator 1.

The actuator 1 may comprise a motor **2,** conveniently an electric one, designed to generate the motion; and a planetary gear train 3 designed to transmit the motion from the motor 2 to the outputs 1a and 1b; and a crankcase 4 housing at least part of the actuation group.

The motor 2 defines a rotary motion along the longitudinal axis 1c.

The planetary gear train 3 comprises, briefly, a sun gear **31** designed to receive the motion from the motor 2; a satellite group 32; a planetary gear train 33 defining the first output 1a; and a crown **34** defining the second output 1b.

It can be epicycloid.

The sun gear 31 may comprise a shaft conveniently having the longitudinal axis 1c as its axis.

The satellite group 32 is kinematically interposed between the entry of the motion in the planetary gear train 3 (the sun gear 31) and the output of the motion from the planetary gear train 3, consisting of the planetary gear train 33 (first output 1a) or in the crown 34 (second output 1b) as described below.

The satellite group 32 may comprise one or more satellites consisting of wheels led (by friction and/or a toothed coupling) by the sun gear 31 and dragging the planetary gear 33 or crown 34.

The planetary gear train 3 is preferably a conveniently cycloid reducer. It can, therefore, as detailed below, exploit one or more cycloid discs (constituting the satellite group 32) in eccentric rotation and having insertion holes for cylinders integral with the planetary gear 33 and having diameters smaller than the cylinders themselves; and outer lobes designed to be engaged with the lobes inside the crown and fewer in number (usually one) than the lobes inside the crown.

The sun gear 31 may comprise a shaft conveniently comprising at least one cam. More preferably, the sun gear 31 may comprise a shaft at at least two cams equally angularly spaced so as to minimise the vibrations owing to its rotation.

Each cam is circular with an axis parallel to and separate from the longitudinal axis 1c.

The sun gear 31 basically has the longitudinal axis 1c as its rotation axis.

The satellite group 32 may comprise at least one cycloidal disk 32a attached and conveniently integral with the sun gear 31. In particular, it may comprise, for each cam of the sun gear 31, a cycloidal disk 32a attached to a cam.

The sun gear 31 may preferably comprise two cams opposite to the longitudinal axis 1c and the satellite group 32 may comprise two cycloidal disks 32a each of which is attached to one of the cams.

The planetary gear 33 is designed to basically rotate around the longitudinal axis 1c.

The planetary gear 33 may comprise a rotating body **33a** defining the first output 1a; connection means for connecting the rotating body 33a to the satellite group 32; and, in some cases, a support **33b** for the rotating body 33b.

The rotating body 33a may comprise a first pulley **33c** including a first throat defining the first output 1a and, in particular, engaging the above-mentioned first transmission cable.

The support 33b is placed on the opposite side to the rotating body 33a in relation to the satellite group 32 along the longitudinal axis 1c and attached integrally to the rotating body 33a by the connection means.

Each cycloidal disk 32a may comprise through holes **32b** and the connection means comprise pins **33d** with a smaller diameter than the through holes 32b. The diameter of the pins 33d is basically less than 70% and, specifically, less than 50% of the diameter of the through holes 32b.

The pins 33d are basically equidistant along the longitudinal axis 1c.

The pins 33d are basically equally angularly spaced apart.

The through holes 32b are basically equidistant from the cam axis.

The through holes 32b are almost equally angularly spaced apart.

The crown 34 is connected to the satellite group 32 and, in particular, to at least one cycloidal disk 32a (conveniently, just one cycloidal disk 32a) so as to be dragged by it.

The transmission ratio between the crown 34 and the satellite group 32 is basically less than 1 and, in particular, basically ranges between 1 and 0.5 and, specifically, between 1 and 0.8.

For this purpose, the crown 34 may comprise, on the inner surface, i.e. facing the satellite group 32, inner lobes **34a** and the cycloidal disk 32a may comprise, on the outer surface, i.e. facing the crown 32, outer lobes **32c,** designed to strike against the inner lobes 34a, pushing and therefore rotating the crown 34.

The number of outer lobes 32c is preferably greater, conveniently by one unit, than the number of inner lobes 34a.

The height of the lobes 32c and/or 34a is such as to enable just a part of the outer lobes 32c to come into contact with the inner lobes 34a.

The crown 34 may comprise a second throat defining the second output 1b and, in particular, engage the above-mentioned second transmission cable.

The crankcase 4 is designed to remain still during the operation of the power assembly 4.

It has a first opening **4a** for accessing the first output 1a and a second opening **4b** for accessing the second output 1b.

The actuator 1 can, in addition, comprise a supplementary planetary gear train **5** placed between the motor 2 and the planetary gear train 3.

The supplementary planetary gear train 5 may mainly comprise a supplementary sun gear **51** designed to receive the motion from the motor 2; a supplementary satellite group **52;** a supplementary differential case **53;** and a supplementary crown **54.**

It can be epicycloid.

In some cases, the supplementary planetary gear train 5 may define a transmission ratio of approximately 3:1.

The supplementary sun gear 51 consists of the output shaft of the motor 2.

The supplementary satellite group 52 may comprise one or more satellites, conveniently four, consisting of wheels designed to be led (by toothed coupling or, preferably, friction so as to limit the maximum torque that can be transmitted) by the supplementary sun gear 51 and drag the supplementary planetary gear 53.

The supplementary crown 54 is integral with the crankcase 4. It therefore remains during the operation of the supplementary planetary gear train 5.

The supplementary planetary gear 53 is moved by the supplementary satellite group 52 and connected, conveniently integrally, with the sun gear 31 that is, thus, led by it.

Finally, it should be noted that the actuator 1 may comprise one or more supports for the planetary gears 3 and/or 5, bushings, bearings, and other similar elements that are represented in the figures but not numbered.

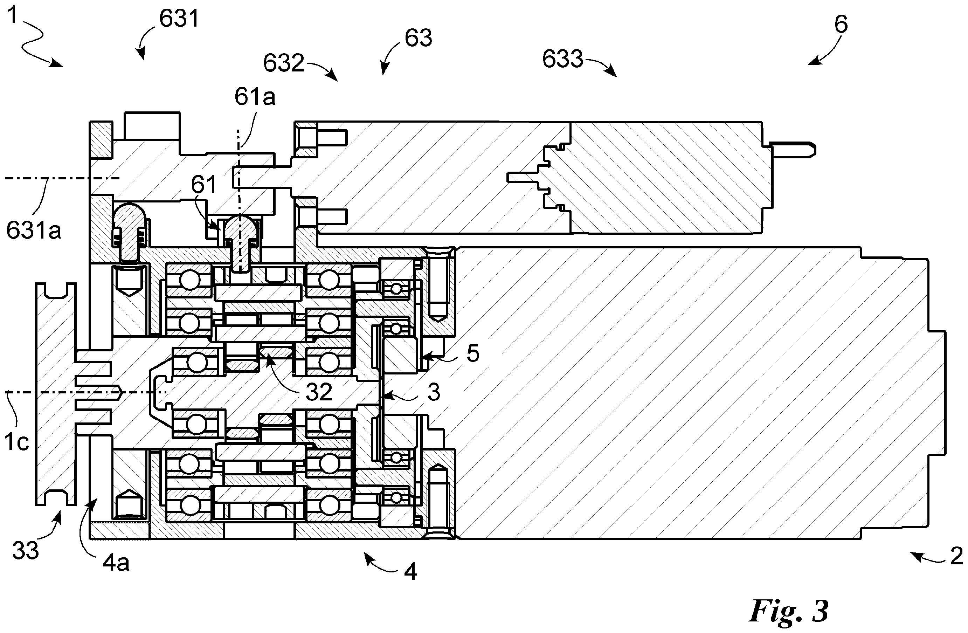

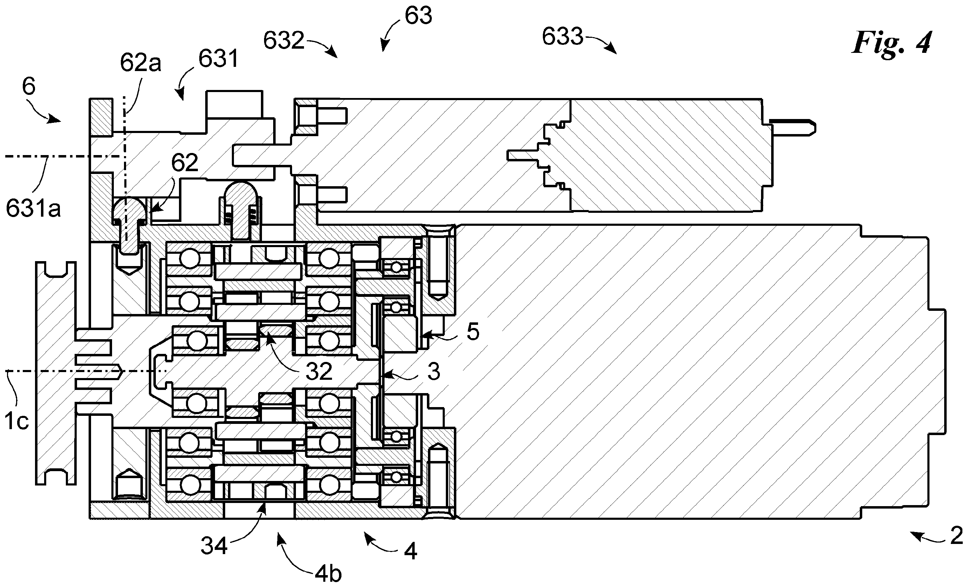

The actuator 1 may comprise a selector 6 designed to selectively lock the rotation of the planetary gear 33 and of the crown 34 by defining a first operating condition (Fig. 3) in which the crown 34 is locked and the robotic device 10 receives the motion and, thus, is moved by the first output 1a and a second operating condition (Fig. 4) in which the planetary gear 33 is locked and the transmission kinematic mechanism 13 receives the motion from the second output 1b. In particular, the selector 6 defines a first operating condition in which the transmission kinematic mechanism 13 receives the motion from the first output 1a and a second operating condition in which the transmission kinematic mechanism 13 receives the motion from the second output 1b.

In the first operating condition, the selector 6 locks the crown 34 and the first output 1a of the actuator 1 is active and, thus, capable of controlling the robotic device 10. In this first operating condition, the selector 6 locks the crown 34 to the crankcase 4.

In the second operating condition, the selector 6 locks the planetary gear 33 and the crankcase 4 has the second output 1b active and, thus, capable of controlling the robotic device 10. In this second operating condition, the selector 6 locks the planetary gear 33 to the crankcase 4.

The actuator 1 is available only in the first configuration or in the second operating configuration. The selector 6 is, thus, designed to only shift between first and second operating configuration. It cannot, therefore, enable the simultaneous locking or moving of the outputs 1a and 1b.

The selector 6 is attached to the crankcase 4.

The selector 6 can comprise a first lock **61** for locking the crown 34; a second lock **62** for locking the planetary gear 33; and a control **63** for activating the locks 61 and 62.

The first lock 61 may comprise a first drum **611** that is integral with the crown 34 and comprising at least one first hole **611a;** a first pin **611** attached to the crankcase 4 and designed to be inserted inside the first hole 611a; a first guide **613** attached to the crankcase 4, defining a first locking axis **61a** and designed to enable the first pin 611 to slide in relation to the crankcase 4 along the first locking axis 61a; and first return means **614** designed to operate against the entry of the first pin 611 in the first hole 611a.

The first lock 61 defines a first active position in which the first pin 611 is partially inserted into the first hole 611a locking the crown 34 to the crankcase 4 and a first inactive position in which the first pin 611 is not even partially inserted into the first hole 611a, enabling the crown 34 to rotate in relation to the crankcase 4.

The first locking axis 61a is basically perpendicular to the longitudinal axis 1c. The first drum 611 may comprise a plurality of first holes 611a equally angularly spaced apart.

The first return means 614 are designed to control the passage of the first pin 611 into a first inactive position. They may comprise a spring, conveniently a compression one.

The second lock 62 may comprise a second drum **621** that is integral with the planetary gear 33 and comprising at least one second hole **621a;** a second pin **622** designed to be inserted into the second hole 621a; a second guide **623** attached to the crankcase 4 defining a second locking axis **62a** and designed to enable the second pin 622 to slide along the second locking axis 62a in relation to the crankcase 4; and second return means **624** designed to operate against the entry of the second pin 622 into the second hole 621a.

The second lock 62 defines a second active position in which the second pin 622 is partially inserted into the second hole 621a, locking the planetary gear 33 to the crankcase 4; and a second inactive position in which the second pin 622 is not even partially inserted into the second hole 621a, enabling the planetary gear 33 to rotate in relation to the crankcase 4.

The second locking axis 62a is basically perpendicular to the longitudinal axis 1c. The second drum 621 may comprise a plurality of second holes 621a equally angularly spaced apart.

The second return means 624 are designed to control the passage of the second pin 622 into the second inactive position. They may comprise a spring, conveniently a compression one.

The control 63 is designed to control the locks 621 and 632 at the same time so that they are always conveniently available, and, therefore, to control the passage of one of the locks 621 and 632 into the active position and the other into the inactive position.

The control 63 may comprise a shaft **631** designed to control the locks 61 and 62 defining a main extension axis **631a;** actuation means **632** for rotating the shaft 631 around the main extension axis 631a; and, conveniently, an encoder **633** for said actuator 632.

The actuation means 632 consist of an electric motor.

The shaft 631 may comprise a first eccentric **631b** to control the passage of the first lock 61 into the first active position and a second eccentric **631c** to control the passage of the second lock 62 into the second active position.

The shaft 631 is designed to rotate around the main extension axis 631a so as to enable each eccentric 631b and 631c to push a pin 611 or 622 into the corresponding hole 611a and 621a in opposition to the return means 614 and 624. The actuator 1 may comprise, finally, a card **7** designed to control the operation of the actuator 1 (i.e. of the robotic device 10) and, in particular, of the selector 6 and of the actuation group.

The operation of the actuator 1 and, in particular, of the robotic device 10 previously described in structural terms is as follows.

Initially, the actuator 1 is in the first operating condition (Fig. 3). Therefore, the first pin 61, locking the crown 34 to the crankcase 4, is in the active position, while the second lock 62, enabling the planetary gear 33 to rotate in relation to the crankcase 4 and the transmission kinematic mechanism 13 to receive the motion from the first output 1a, is in the second, inactive position.

In the first operating condition, the first eccentric 631b pushes the first pin 611, partially inserting it into the first hole 611a in opposition to the first return means 614, while the second eccentric 631c does not push the second pin 622 that, pushed by the second return means 624, is external to the second hole 621a.

This configuration enables the output motion from the motor 2 to move, conveniently through the supplementary planetary gear train 5, the sun gear 31 that activates the satellite group 32. In turn, the satellite group 32, since the crown 34 is integral with the crankcase 4, rotates the planetary gear 33. The planetary gear 33 rotates, thus, the first output 1a that, through the first transmission cable, activates the transmission kinematic mechanism 13, forcing the under-implemented hand 1 to activate a pose of the robotic device 10.

When the user decides, for example, to perform a different pose with the robotic device 10, he or she controls the passage of the actuator 1 into the second operating condition (Fig. 4) through a myoelectric sensor, or another mechanism designed to send the command to the card 7.

The card 7 makes the shaft 631 rotate about the main extension axis 631a so as to move the first eccentric 631b away from the first lock 61 and bring the second eccentric 631c against the second lock 62.

As a result, the first return means 614 extract the first pin 611 from the first hole 611a, bringing the first lock 61 into the first inactive position and leaving the crown 34 free to rotate in relation to the crankcase 4. At the same time, the second eccentric 631c pushes, in opposition to the second return means 624, the second pin 622 into the second hole 621a bringing the second lock 62 into the active position and, thus, locking the planetary gear 33 to the crankcase 4.

At this point, the actuator 1 is in the second operating configuration. Thus, the motor 2 can move, conveniently through the supplementary planetary gear train 5, the sun gear 31 that activates the satellite group 32. In turn, the satellite group 32, since the planetary gear 33 is integral with the crankcase 4, controls the rotation of the crown 34 and, thus, the second output 1b. As a result, the second output 1b, through the second transmission cable, activates the transmission kinematic mechanism 13, controlling the robotic device 10.

Initially, the actuator 1 is in the first operating condition. Therefore, the first pin 61, locking the crown 34 to the crankcase 4, is in the active position, while the second lock 62 is in the second inactive position, enabling the planetary gear 33 to rotate in relation to the crankcase 4 and, thus, enabling the transmission kinematic mechanism 13 to receive the motion from the first output 1a.

Specifically, in the first operating condition, the first eccentric 631b pushes the first pin 611, that is, thus, partially inserted into the first hole 611a in opposition to the first return means 614, while the second eccentric 631c does not push the second pin 622 that is, thus, pushed by the second return means 614 external to the second hole 621a.

This configuration enables the output motion from the motor 2 to move, conveniently through the supplementary planetary gear train 5, the sun gear 31 that moves the satellite group 32. In turn, the satellite group 32, since the crown 34 is integral with the crankcase 4, controls the rotation of the planetary gear 33. The planetary gear 33 rotates, thus, the first output 1a that, through the first transmission cable, activates the transmission kinematic mechanism 13.

The actuator 1 and, thus, the robotic device 10 according to the invention, entail important advantages.

In fact, unlike currently known hands, the actuator 1, thanks to the presence of two outputs 1a and 1b activated by the same motor 2, makes it possible to obtain a robotic device 10 (conveniently anthropomorphic or non-anthropomorphic) that is more adaptable to the various use needs, notwithstanding the extremely reduced size and weight of the robotic device 10.

This aspect translates, thus, into a simplicity both of construction and, in particular, of control that, not requiring additional encoders or sensors, define a robotic device 10 (for example a hand) that is very comfortable to use and, thus, totally satisfactory in terms of the user's expectations.

Another advantage is the fact that the actuator 1 makes it possible to use the actuation group remotely; the group can, thus, be placed in the most advantageously free point every time and, thus, have motors better adapted to the application.

One advantage is the fact that the actuator 1 enables the outputs 1a and 1b to be activated in both rotation directions, broadening the usability of the robotic device 10.

Another advantage is defined by the detail of the planetary gear train 3 that, since it is composed of an irreversible cycloid system, simplifies the system for selecting the output 1a and 1b.

The invention is susceptible to variations falling within the scope of the inventive concept defined by the claims.

For example, the planetary train 3 may consist of a harmonic drive.

## Claims

1. An actuator (1) designed to control a robotic device (10) and defining a first output (1a) and a second output (1b); said outputs (1a, 1b) being designed to control the motion of said robotic device (10); said actuator (1) comprising
- a motor (2) designed to generate motion;
- a planetary gear train (3) comprising
∘ a sun gear (31) designed to receive motion from said motor (2),
∘ a satellite group (32),
∘ a planetary gear (33) defining said first output (1a) and
∘ a crown (34) defining said second output (1b);
- a selector (6) designed to selectively lock the rotation of said planetary gear (33) and of said crown (34) defining a first operating condition wherein said crown (34) is locked and said robotic device (10) is moved by said first output (1a) and a second operating condition wherein said planetary gear (33) is locked and said robotic device (10) is moved by said second output (1b);
**characterized in that**
- said actuation group comprises a crankcase (4); and wherein said selector (6) comprises: a first lock (61) for locking said crown (34) to said crankcase (4); a second lock (62) for locking said planetary gear (33) to said crankcase (4); and a control (63) for activating said locks (61, 62);
**and in that**
- said first lock (61) comprises a first drum (611) attached to said crown (34) and comprising at least a first hole (611a); a first pin (611) designed for being inserted in said first hole (611a); a first guide (613) designed to allow said first pin (611) to slide with respect to said crankcase (4); and first return means (614) designed to work in opposition to the entry of said first pin (611) in said first hole (611a).

2. The actuator (1) according to claim 1, wherein said sun gear (31) comprises at least one cam; and wherein said satellite group (32) comprises at least one cycloidal disk (32a) axially attached to said cam.

3. The actuator (1) according to claim 2, wherein said sun gear (31) comprises two equally angularly spaced cams; and wherein said satellite group (32) comprises two cycloidal disks (32a) each of which is attached to one of said cams.

4. The actuator (1) according to at least one of claims 2-3, wherein said at least one cycloidal disk (32a) comprises through-holes (32b); wherein said planetary gear (33) comprises a rotating body (33a) defining said first output (1a) and pins (33d) for engaging said rotating body (33a) to said satellite group (32); and wherein the diameter of said pins (33d) is less than the diameter of said through-holes (32b).

5. The actuator (1) according to at least one previous claim, wherein said crown (34) comprises inner lobes (34a) on the inner surface; wherein said at least one cycloidal disc (32a) comprises outer lobes (32c) on the outer surface that are designed to contrast with said inner lobes (34a); and wherein the number of said outer lobes (32c) is lower than the number of said inner lobes (34a).

6. The actuator (1) according to claim 1, wherein said second lock (62) comprises a second drum (621) attached to said planetary gear (32) and comprising at least one second hole (621a); one second pin (622) designed for being inserted in said second hole (621a); a second guide (623) designed to allow said second pin (622) to slide with respect to said crankcase (4); and second return means (624) to work in opposition to the entry of said second pin (622) in said second hole (621a).

7. A robotic device (10) comprising at least one actuator (1), according to one or more of the previous claims, and a transmission kinematic mechanism (13) designed to control the actuation of said robotic device (10) on the basis of the output motion from said actuator (1); and wherein in said first operating condition, said transmission kinematic mechanism (13) receives said motion from said first output (4a) and a second operating condition wherein said transmission kinematic mechanism (13) receives said motion from said second output (4b).

8. The robotic device (10) according to the previous claim, wherein said robotic device (10) is a manipulator.

9. The robotic device (10) according to the previous claim, wherein said robotic device (10) is a robotic hand.

## Patentansprüche

1. Aktuator (1), der ausgelegt ist, eine Robotervorrichtung (10) zu steuern und der einen ersten Ausgang (1a) sowie einen zweiten Ausgang (1b) definiert; wobei die Ausgänge (1a, 1b) ausgelegt sind, die Bewegung der Robotervorrichtung (10) zu steuern; wobei der Aktuator (1) umfasst:
- einen Motor (2), der ausgelegt ist, eine Bewegung zu erzeugen;
- ein Planetengetriebezug (3) umfassend
∘ ein Sonnenrad (31), das ausgelegt ist, Bewegung von dem Motor (2) aufzunehmen,
∘ eine Satellitengruppe (32),
∘ ein Planetengetriebe (33), das den ersten Ausgang (1a) definiert, und
∘ einen Kranz (34), der den zweiten Ausgang (1b) definiert;
- einen Wähler (6), der ausgelegt ist, wahlweise die Drehung des Planetengetriebes (33) und des Kranzes (34) zu sperren, indem ein erster Betriebszustand definiert wird, wobei der Kranz (34) gesperrt wird und die Robotervorrichtung (10) durch den ersten Ausgang (1a) bewegt wird, und ein zweiter Betriebszustand, wobei das Planetengetriebe (33) gesperrt wird und die Robotervorrichtung (10) durch den zweiten Ausgang (1b) bewegt wird;
**dadurch gekennzeichnet, dass**
- die Betätigungsgruppe ein Kurbelgehäuse (4) umfasst; und wobei der Wähler (6) ein erstes Sperrelement (61) zum Sperren des Kranzes (34) an dem Kurbelgehäuse (4); ein zweites Sperrelement (62) zum Sperren des Planetengetriebes (33) an dem Kurbelgehäuse (4); und eine Steuerung (63) zum Aktivieren der Sperrelementen (61, 62) umfasst;
**und dass**
- das erste Sperrelement (61) eine erste Trommel (611) umfasst, die mit dem Kranz (34) befestigt ist und umfassend mindestens eine erste Bohrung (611a); einen ersten Stift (611), der ausgelegt ist, in die erste Bohrung (611a) eingeführt zu werden; eine erste Führung (613), die ausgelegt ist, dem ersten Stift (611) zu ermöglichen, in Bezug auf das Kurbelgehäuse (4) zu gleiten; und erste Rücklaufmittel (614), die ausgelegt sind, in Opposition zu dem Eintritt des ersten Stifts (611) in die erste Bohrung (611a) zu wirken.

2. Aktuator (1) nach Anspruch 1, wobei das Sonnenrad (31) mindestens eine Nocke umfasst; und wobei die Satellitengruppe (32) mindestens eine Zykloidscheibe (32a) umfasst, die axial an der Nocke befestigt wird.

3. Aktuator (1) nach Anspruch 2, wobei das Sonnenrad (31) zwei gleichwinklig beabstandete Nocken umfasst; und wobei die Satellitengruppe (32) zwei Zykloidscheiben (32a) umfasst, von denen jede an einer der Nocken befestigt wird.

4. Aktuator (1) nach mindestens einem der Ansprüche 2-3, wobei die mindestens eine Zykloidscheibe (32a) Durchgangsbohrungen (32b) umfasst; wobei das Planetengetriebe (33) einen Drehkörper (33a) umfasst, der den ersten Ausgang (1a) definiert, und Stifte (33d) zum Befestigen des Drehkörpers (33a) an der Satellitengruppe (32); und wobei der Durchmesser der Stifte (33d) kleiner als der Durchmesser der Durchgangsbohrungen (32b) ist.

5. Aktuator (1) nach mindestens einem vorhergehenden Anspruch, wobei der Kranz (34) innere Vorsprunge (34a) auf der inneren Oberfläche umfasst; wobei die mindestens eine Zykloidscheibe (32a) äußere Vorsprunge (32c) auf der äußeren Oberfläche umfasst, die ausgelegt sind, zu den inneren Vorsprungen (34a) in Kontrast zu stehen; und wobei die Anzahl der äußeren Vorsprunge (32c) kleiner als die Anzahl der inneren Vorsprunge (34a) ist.

6. Aktuator (1) nach Anspruch 1, wobei das zweite Sperrelement (62) eine zweite Trommel (621) umfasst, die an dem Planetengetriebe (33) befestigt ist und mindestens eine zweite Bohrung (621a) umfasst; einen zweiten Stift (622), der ausgelegt ist, in die zweite Bohrung (621a) eingeführt zu werden; eine zweite Führung (623), die ausgelegt ist, dem zweiten Stift (622) zu ermöglichen, in Bezug auf das Kurbelgehäuse (4) zu gleiten; und zweite Rücklaufmittel (624), um in Opposition zu dem Eintritt des zweiten Stifts (622) in die zweite Bohrung (621a) zu wirken.

7. Robotervorrichtung (10) umfassend mindestens einen Aktuator (1) nach einem oder mehreren der vorhergehenden Ansprüche und einen kinematischen Übertragungsmechanismus (13), der ausgelegt ist, die Betätigung der Robotervorrichtung (10) basierend auf der Ausgangsbewegung des Aktuators (1) zu steuern und wobei in dem ersten Betriebszustand der kinematische Übertragungsmechanismus (13) die Bewegung von dem ersten Ausgang (4a) empfängt und in einem zweiten Betriebszustand wobei der kinematische Übertragungsmechanismus die Bewegung von dem zweiten Ausgang (4b) empfängt.

8. Robotervorrichtung (10) nach dem vorhergehenden Anspruch, wobei die Robotervorrichtung (10) ein Manipulator ist.

9. Robotervorrichtung (10) nach dem vorhergehenden Anspruch, wobei die Robotervorrichtung (10) eine Roboterhand ist.

## Revendications

1. Un actionneur (1) conçu pour commander un dispositif robotique (10) et définissant une première sortie (1a) et une deuxième sortie (1b); lesdites sorties (1a, 1b) étant conçues pour commander le mouvement dudit dispositif robotique (10); ledit actionneur (1) comprenant:
- un moteur (2) conçu pour générer un mouvement;
un train d'engrenage planétaire (3) comprenant:
∘ un pignon solaire (31) conçu pour recevoir le mouvement dudit moteur (2),
∘ un groupe satellite (32),
∘ un engrenage planétaire (33) définissant ladite première sortie (1a), et
∘ une couronne (34) définissant ladite deuxième sortie (1b);
- un sélecteur (6) conçu pour bloquer sélectivement la rotation dudit engrenage planétaire (33) et de ladite couronne (34), définissant une première condition de fonctionnement dans laquelle ladite couronne (34) est bloquée et ledit dispositif robotique (10) est actionné par ladite première sortie (1a), et une deuxième condition de fonctionnement dans laquelle ledit engrenage planétaire (33) est bloqué et ledit dispositif robotique (10) est actionné par ladite deuxième sortie (1b);
**caractérisé en ce que**
- ledit groupe d'actionnement comprend un carter (4); et **en ce que** ledit sélecteur (6) comprend : un premier verrou (61) pour bloquer ladite couronne (34) audit carter (4); un deuxième verrou (62) pour bloquer ledit engrenage planétaire (33) audit carter (4); et un mécanisme de commande (63) pour activer lesdits verrous (61, 62);
**et en ce que**
- ledit premier verrou (61) comprend un premier tambour (611) fixé à ladite couronne (34) et comprenant au moins un premier trou (611a); une première broche (611) conçue pour être insérée dans ledit premier trou (611a); un premier guide (613) conçu pour permettre à ladite première broche (611) de coulisser par rapport audit carter (4); et des premiers moyens de rappel (614) conçus pour s'opposer à l'entrée de ladite première broche (611) dans ledit premier trou (611a).

2. L'actionneur (1) selon la revendication 1, dans lequel ledit pignon solaire (31) comprend au moins une came; et dans lequel ledit groupe satellite (32) comprend au moins un disque cycloïdal (32a) fixé axialement à ladite came.

3. L'actionneur (1) selon la revendication 2, dans lequel ledit pignon solaire (31) comprend deux cames également espacées angulairement; et dans lequel ledit groupe satellite (32) comprend deux disques cycloïdaux (32a), chacun étant fixé à l'une desdites cames.

4. L'actionneur (1) selon au moins l'une des revendications 2-3, dans lequel ledit au moins un disque cycloïdal (32a) comprend des trous traversants (32b); dans lequel ledit engrenage planétaire (33) comprend un corps rotatif (33a) définissant ladite première sortie (1a) et des broches (33d) permettant de fixer ledit corps rotatif (33a) audit groupe satellite (32); et dans lequel le diamètre desdites broches (33d) est inférieur au diamètre desdits trous traversants (32b).

5. L'actionneur (1) selon au moins l'une des revendications précédentes, dans lequel ladite couronne (34) comprend des lobes internes (34a) sur la surface intérieure; dans lequel ledit au moins un disque cycloïdal (32a) comprend des lobes externes (32c) sur la surface extérieure conçus pour contraster avec lesdits lobes internes (34a); et dans lequel le nombre desdits lobes externes (32c) est inférieur au nombre desdits lobes internes (34a).

6. L'actionneur (1) selon la revendication 1, dans lequel ledit deuxième verrou (62) comprend un deuxième tambour (621) fixé audit engrenage planétaire (32) et comprenant au moins un deuxième trou (621a); une deuxième broche (622) conçue pour être insérée dans ledit deuxième trou (621a); un deuxième guide (623) conçu pour permettre à ladite deuxième broche (622) de coulisser par rapport audit carter (4); et des deuxièmes moyens de rappel (624) pour s'opposer à l'entrée de ladite deuxième broche (622) dans ledit deuxième trou (621a).

7. Un dispositif robotique (10) comprenant au moins un actionneur (1) selon une ou plusieurs des revendications précédentes, et un mécanisme cinématique de transmission (13) conçu pour commander l'actionnement dudit dispositif robotique (10) en fonction du mouvement de sortie dudit actionneur (1); et dans lequel, dans ladite première condition de fonctionnement, ledit mécanisme cinématique de transmission (13) reçoit ledit mouvement de ladite première sortie (4a), et dans une deuxième condition de fonctionnement, ledit mécanisme cinématique de transmission (13) reçoit ledit mouvement de ladite deuxième sortie (4b).

8. Le dispositif robotique (10) selon la revendication précédente, dans lequel ledit dispositif robotique (10) est un manipulateur.

9. Le dispositif robotique (10) selon la revendication précédente, dans lequel ledit dispositif robotique (10) est une main robotique.
